# EUROPEAN PATENT SPECIFICATION

(11) **EP 1 620 193 B1**
(45) Date of publication and mention of the grant of the patent: **03.09.2008**
(21) Application number: 04729890.6
(22) Date of filing: 28.04.2004
(51) Int. Cl.: B01D 9/00

(54) **ANTISOLVENT SOLIDIFICATION PROCESS**
VERFAHREN ZUR VERFESTIGUNG, BEI WELCHEM EIN ANTI-LÖSUNGSMITTEL BENUTZT WIRD
PROCEDE DE SOLIDIFICATION EN EMPLOYANT UN ANTISOLVANT

(30) Priority: 29.04.2003 US 466761 P
(43) Date of publication of application: 01.02.2006
(73) Proprietor: N.V. Organon, 5349 AB Oss (NL)
(72) Inventor: BAKKER, Wridzer, Jan, Willem, NL-6821 EG Arnhem (NL); GEERTMAN, Robert, Michael, NL-6836 PL Arnhem (NL); REEDIJK, Marianne, Frederika, NL-7328 WS Apeldoorn (NL); BALTUSSEN, Jozef, Johannes, Maria, NL-6531 PE Nijmegen (NL); BARGEMAN, Gerrald, NL-6708 NW Wageningen (NL); VAN LARE, Cornelis, Elizabeth, Johannus, NL-6602 GE Wijchen (NL)
(74) Representative: Broekkamp, Chris L. E.
(86) International application number: PCT/EP2004/004506
(87) International publication number: WO 2004/096405

(56) References cited:
- WO-A-03/032951
- US-B1- 6 221 398

## Description

### TECHNICAL FIELD

The present invention relates to a process for the solidification of inorganic or organic compounds using a novel antisolvent solidification technique.

### BACKGROUND OF THE INVENTION

In industry, solidification is an often used technique for the purification of inorganic or organic compositions, since in general, solidification requires lower energy than other separation processes. Most of the industrial applications involve solidification of a compound from a solution by directly or indirectly cooling said solution and/or by evaporating part of the solvent in order to effect solidification. For example, many inorganic salts are made industrially from aqueous solutions which are produced by dissolving a natural source of the salt in water. The salt is usually obtained by crystallising it from the aqueous solution by evaporation of the water, which is generally accomplished using multiple-effect or vapour recompression evaporators. However, such evaporation processes are energy intensive. If the separation of a salt from water could be done without vaporising water, substantial energy savings would be possible.

Another method for the solidification of inorganic or organic compositions is by an antisolvent solidification process. In an antisolvent process the compound which is to be solidified is obtained by the addition of an antisolvent to a solvent comprising said compound, or vice versa. In this way the solidification of the compound is induced. Subsequently, if so desired, the obtained compound may be filtered from the reaction mixture. When the compound is precipitated as crystals, the method is generally referred to as antisolvent crystallisation.
Especially for the production of inorganic salts, antisolvent crystallisation can be an energy saving alternative for the generally employed evaporative crystallisation processes. However, a general drawback of such antisolvent methods is that due to the high supersaturations involved, impurities tend to precipitate together with the product. Also, in prior art antisolvent crystallisation, the occurrence of agglomerates or morphological Instabilities is often observed, since these growth forms are sensitive to mother liquor entrapment. Inside the agglomerates, the voids will be filled with mother liquor. Therefore, additional washing steps or recrystallisations are usually needed in order to obtain a product with the desired purity.
Another drawback of conventional antisolvent processes is that the particle size of the obtained products may vary widely, due to the geometry of the vessel and the speed and location of addition of the antisolvent. For example, when scaling up such a process, or when changes in the process set-up are made, a different product might be obtained because the process circumstances have changed. Especially for processes on an industrial scale, the lack of reproducibility and robustness of such an antisolvent process can be problematic.
For many industrial applications, the particle size of the solidified compounds is very important, since it influences *int. al.* the rate of dissolution and the storage stability of a compound. Hence, trying to control the eventual particle size of a compound has been the subject of many research topics. This issue is of particular interest in the area of pharmaceutical product development. Particle size distribution is important when one wants to obtain pharmaceutically acceptable products, for example in respect of content uniformity in the final dosage forms and in respect of rate of dissolution of solid dosage forms. For example, in low dose dosage forms it might be difficult to obtain a good homogeneity if the particle size is too large. Moreover, a large particle size can make the pharmaceutical compound difficult to process into a pharmaceutical end product. For example, the particle size also influences the ease of segregation in a mixing process, which takes place prior to tabletting.
For the same reasons the controllability of the particle size is important. For instance, a wide variation in particle size of the pharmaceutical compound can lead to insufficient control of the concentration of the pharmaceutical compound in the pharmaceutical end product. Furthermore, a wide variation in particle size or a large particle size of the pharmaceutical compound might necessitate an extra micronisation or milling step.
Often particles of pharmaceutical compounds are preferably prepared in a crystalline form. If such a pharmaceutical compound is prepared in a crystalline form, the purity, crystal size distribution, and polymorphy of the crystals can be very important. For example, differences in crystal structure can lead to a difference in physico-chemical parameters such as stability, rate of dissolution, melting point, analytical data and the like, which frequently are strongly influenced by the crystal forms of a polymorphous compound.

A method often used in antisolvent crystallisation is the so-called Quasi-Emulsion Solvent Diffusion (QESD) method. This is for instance described in "Organic Particle Precipitation" by J. Texter (Reactions and Synthesis in Surfactant Systems (Marcel Dekker 2001), pp. 577 - 607). In the QESD method, droplets of solvent with dissolved crystalline material are generated in an antisolvent. Typically, the droplets are generated via high shear methods, a technique well-known in the art of mixing. Once these droplets are formed, the antisolvent diffuses into the droplets, leading to precipitation of the crystals, *i.e.* the solvent and the antisolvent need to diffuse out of and into the droplets, respectively. The crystals formed are dispersed in the mixture of antisolvent and solvent (which is diffused out of the original droplets). If required, emulsifier (or a mixture of emulsifiers) can be added to the antisolvent and/or solvent to help stabilise the droplets. The key to this process, however, is that the droplets are formed where the antisolvent solution acts as the continuous phase. This is for instance described by M. Nocent et al. in J. of Pharmaceutical Sciences, Vol. 90, No.10, October 2001, p. 1620. In the QESD method it is tried to achieve control over the eventual crystal size by tuning the employed mixing energy, as this controls the droplet size. At the same time the droplet size is governed by the physical interaction between the solvent and the antisolvent, as this is controlled by for instance the surface tension. However, due to the fact that in the QESD method the emulsification and the antisolvent crystallisation occur simultaneously, the particle size distribution of the compound which is solidified is very difficult to control.

WO 90/03782 describes a process for producing finely divided solid crystalline or amorphous powders. Said process comprises the steps of dissolving a solid in a liquid carrier solvent to form an injection solution and adding this solution to a compressed liquefied or supercritical gas atmosphere, which gas is essentially an antisolvent or nonsolvent for the solid to be micronised or subdivided as a solid.
H. Kroeber et al. in 15th International Symposium on Industrial Cystallisation, 15th, Sorrento Italy, September 2002, describe a process for the preparation of fine particles by precipitation with a compressed fluid antisolvent. For instance, liquid solutions of tartaric acid in acetone, ethanol, and methanol/ethanol mixtures are sprayed via a nozzle into supercritical carbon dioxide used as antisolvent.
F. Espitalier et al., 12th Symposium on Industrial Crystallisation, September 1993, Vol. 1, pp. 25-31, describe a process wherein a drug is dissolved in a solvent at high temperature and introduced through a capillary of variable diameter into a nonsolvent at a lower temperature.
WO 00/38811 describes a process for preparing crystalline particles comprising the mixing in the presence of ultrasonic radiation of a flowing solvent solution of a substance in a liquid solvent with a flowing liquid antisolvent for said substance.
These prior art antisolvent processes, however, are known to be notoriously difficult to control. It is difficult to scale up to a higher volume and/or to obtain robust control of the particle size.

For the above-mentioned reasons there is a need for an improved antisolvent process, one which is less energy consuming than conventional evaporative processes and which provides a solidified compound of the desired quality and size. Moreover, there is a need for an improved antisolvent crystallisation process wherein the particle size distribution can be controlled.

Surprisingly, we have now found that by using a membrane for the dosing of an antisolvent to a liquid medium comprising a dissolved organic or inorganic compound or vice versa, an improved antisolvent solidification process is obtained. More particularly, by using a membrane as a precision dosing device, a controlled solidification, preferably crystallisation, process is obtained, yielding a composition comprising solid particles comprising said organic or inorganic compound which are in general non-agglomerated and which have an improved quality (e.g. an improved shelf life). Moreover, with the process according to the invention preferably higher product yields are obtained and less off-specs. In other words, the antisolvent solidification process according to the present invention comprises a more efficient use of (starting) materials and/or solvents and therefore a decrease in energy consumption can be achieved compared to conventional evaporative or antisolvent processes. Furthermore, the process can easily be scaled up to a higher volume and enables a robust control of the particle size.

The use of membranes in the distant field of emulsion technology is known. R. William et al., for example, in Chemical Engineering Research and Design (Part A) Vol. 76, 1998, pp. 894-901 and 902-910, describe the controlled production of emulsions using a crossflow membrane. Emulsions are produced by breaking up a discontinuous phase through a porous membrane. Subsequently, the droplets formed on the surface of the other side of the membrane are scoured away by the crossflow of a continuous phase.

The use of membranes in an evaporative crystallisation process is described by E. Curcio et al. in Ind. Eng. Chem. Res. 2001, Vol. 40, pp. 2679-2684. Here a so-called membrane distillation technique is combined with an evaporative crystallisation process. More precisely, a microporous hydrophobic membrane is in contact with a hot feed on one side and with a condensing solution on the other. At both layers of the membrane, a vapour-liquid equilibrium is established, giving rise to a vapour pressure gradient. The solvent evaporation occurs inside the membrane module where the flowing solution is below the supersaturation condition, but the crystallisation stage is performed in a separate tank.
In addition, J. Zhiquian et al. in their articles "Synthesis of Nanosized BaSO4 Particles with a Membrane Reactor: Effects of Operating Parameters on Particles," Journal of Membrane Science Vol. 209, 2002, pp. 153-161 and "Synthesis of Nanosized BaSO4 and CaCO3 Particles with a Membrane Reactor: Effects of Additives on Particles," Journal of Colloid and Interface Science Vol. 266, 2003, pp. 322-327, describe the preparation of BaSO₄ particles by adding a Na₂SO₄ solution to a BaCl₂ solution through a membrane. The desired compound was only formed after the combination and reaction of the two solutions. In the second article, furthermore, the effects of very small amounts of additives such as ethanol, acetone, and acetic acid on the particles' morphology were explored. For example, particles were prepared in the presence of up to 0.1713 mol l⁻¹ ethanol. It was mentioned that these additives can adsorb on steps and kinks of the particle surface, leading to Inhibition of particle growth. Because of the low concentrations, the additives cannot act as either a solvent or an antisolvent.

US 6 221 398 B1 describes a process for producing a pharmaceutical powder for inhalation comprising crystalline particles of an inhalation compound, comprising dissolving an inhalation compound in a solvent; and introducing the solution containing the inhalation compound into an anti-solvent through a porous filler having pores of 10-160 microns, such as Pyrex Glass Filters.

### SUMMARY OF THE INVENTION

Accordingly, the present invention provides an antisolvent solidification process wherein a liquid medium comprising at least one dissolved organic or inorganic compound is forced through a membrane which is positioned in a membrane module into one or more antisolvents or wherein one or more antisolvents are forced through a membrane which is positioned in a membrane module into a liquid medium comprising at least one organic or inorganic compound, whereby the process is carried out as a continuous process, and whereby the membrane has up to 3 µm pores and the shape of the membrane is selected from tubes, fibres, or spiral wounds, yielding a composition comprising solid particles comprising said organic and/or inorganic compound(s).
It was surprisingly found that by using the membrane as a precision dosing device, efficient micromixing of the liquid medium comprising at least one dissolved compound and the one or more antisolvents can be achieved. In this manner, local variations in supersaturation, which in conventional antisolvent solidifications are often responsible for the uncontrolled precipitation of solids and the formation of strongly agglomerated particles, can be avoided. Hence, no or significantly fewer agglomerated solid particles will be formed using this novel solidification process.

### THE FIGURES

Figure 1 shows a SEM photo of sodium chloride crystals obtained according to the procedure as described in Example 1.
Figure 2A shows a SEM photo of 3-ketodesogestrel crystals obtained according to the procedure as described in Example 2, whereas Figure 2B shows a SEM photo of 3-ketodesogestrel crystals obtained according to the procedure as described in Comparative example 3.

### DETAILED DESCRIPTION OF THE INVENTION

The process according to the present invention is a novel antisolvent solidification process wherein a liquid medium comprising at least one dissolved organic or inorganic compound is forced through a membrane which is positioned in a membrane module into one or more antisolvents or wherein one or more antisolvents are forced through a membrane which is positioned in a membrane module into a liquid medium comprising at least one dissolved organic or inorganic compound, whereby the process is carried out as a continuous process, and whereby the membrane has up to 3 µm pores and the shape of the membrane is selected from tubes, fibres, or spiral wounds, yielding a composition comprising solid particles comprising said organic and/or inorganic compound(s). By using the membrane as a precision dosing device, efficient micromixing of the antisolvent(s) and the liquid medium comprising the compound(s) which is/are to be solidified can be achieved while forming a solution or, in a less preferred embodiment, an emulsion, from which the compound(s) will solidify, preferably crystallise, into solid, preferably crystalline, particles.
Due to said controlled mixing, the process of the present invention results in much more controlled solidification conditions, due for example to better control of hydrodynamics, temperature, and (local) concentrations. When the solidification is a crystallisation, a composition comprising crystalline particles with a more uniform morphology, higher purity, and less attrition compared to conventional crystallisation techniques can be obtained, while the process is more flexible than conventional processes.

Compounds suitable for being solidified via the process according to the present invention can for example be organic compounds such as pharmaceutical or technical chemicals, or inorganic compounds such as alkali or alkaline earth metal salts or heterogeneous catalysts or catalyst intermediates or catalyst additives. Preferably, the organic or inorganic compound is selected from the group consisting of transition metal compounds, transition metal salts, alkali salts, alkali earth salts, chelating compounds, fatty acids proteins, cellulose derivatives, surfactants, silicates, chlorates, alkali or alkaline earth salts of carboxylic acids, saccharides, aminoacids, and pigments. The solidification process according to the present invention can be used to prepare a composition comprising solid particles comprising organic and/or Inorganic compounds, wherein said solid particles are either amorphous or crystalline. Amorphous particles are particles which have no crystal structure (see Webster's 3rd New International Dictionary, Merriam-Webster Inc., 1993, p. 72), whereas crystalline particles are precipitates of solid matter in which the individual molecules are ordered in a regular pattern within crystalline domains. These particles generated are composed of crystals or fragments of crystals. Such crystals can be monomorphous, i.e. consisting of only one (poly)morphic form, or an isomorphous mixture (see Webster's 3rd New International Dictionary, Merriam-Webster Inc., 1993, p. 72), i.e. comprising more than one (poly)morphic form. In the latter case, the several polymorphs in the crystal can be separated by amorphous regions. In a preferred embodiment the crystals are monomorphous, i.e. they only consist of one (poly)morphic form.

In one preferred embodiment, the organic and/or inorganic compound(s) which is/are to be solidified using the antisolvent process according to the present invention are pharmaceutical compounds. By pharmaceutical compounds are meant compounds which can be used in the treatment of the human or animal body by surgery or therapy or in diagnostic methods practised on the human or animal body, including compounds used in prophylactic therapy and compounds used in contraception. In one further embodiment also intermediates to such pharmaceutical compounds are considered pharmaceutical compounds. Generally, pharmaceutical compounds require authorisation of the appropriate authorities before they can be marketed as a medicine in a specific country.
The pharmaceutical compound can for example be present as free base, its corresponding ester, or as a pharmaceutically acceptable salt. Examples of pharmaceutically acceptable salts include for example maleates, chloride or bromide salts, acetates, sulfates, phosphates, nitrates or propionates. In a preferred embodiment the pharmaceutical compound is chosen from the group of steroid hormones, such as tibolone ((7alfa, 17alfa)-17-hydroxy-7-methyl-19-nor-pregn-5(10)-en-20-yn-3-one), progesterone (pregn-4-ene-3,20-dione), desogestrel (17alfa)-13-ethyl-11-methylene-18,19-dinorpregn-4-en-20-yn-17-ol), and 3-keto-desogestrel (etonogestrel or (17alfa)-17-hydroxy-13-ethyl-11-methylene-18,19-dinorpregn-4-en-20-yn-3-one).

Preferably, the solid particles prepared by the process according to the invention comprise at least one pharmaceutical compound, but they may comprise two or more pharmaceutical compounds or a combination of one or more pharmaceutical compounds and another compound which is not pharmaceutically active. In one embodiment the solid particles essentially consist of particles of only one pharmaceutical compound. In another embodiment the solid particles comprise a mixture of two or more compounds, of which preferably at least one is a pharmaceutical compound. In a further embodiment the solid particles comprise a mixture of two or more pharmaceutical compounds.

The term antisolvent as used throughout this specification is meant to denominate any liquid, supercritical composition or gaseous composition which differs in chemical composition from the liquid medium employed in the process according to the present invention and which, after being mixed at 20°C in a 1:1 mol% ratio with said liquid medium comprising a dissolved compound which is to be solidified, will lower the solubility of said compound to such an extent that within 24 hours, preferably within 12 hours, more preferably within 1 hour, even more preferably within 15 min, and most preferably within 2 min after mixing, at least 5wt% of the dissolved compound, preferably at least 15wt%, and most preferably at least 25wt%, will have solidified. Preferably, the antisolvent is a liquid composition.

To achieve this by using a liquid composition, the solubility of the compound in the antisolvent will preferably be substantially lower than in the liquid medium. Preferably, the amount of compound which is dissolved in a saturated solution of said compound in the antisolvent is at least 10wt% less than the amount dissolved in a saturated solution of said compound in the employed liquid medium, at a temperature of 20°C. Preferably, the amount of dissolved compound is at least 30wt% less, and most preferably the amount is at least 50wt% less than the amount dissolved in a saturated solution of said compound in the employed liquid medium. In addition, the presence of the antisolvent in the mixture of liquid medium, compound to be solidified, and antisolvent preferably reduces the solubility of said compound in said mixture to such an extent that even after correction for the increased volume of the resulting mixture after the addition of the antisolvent to the liquid medium, the total amount of compound to be solidified which is dissolved in said mixture is lower than the amount of said compound which was dissolved in the liquid medium before the addition of the antisolvent. This can be illustrated with the following, non-binding example: 100 g of a compound are dissolved in 1,000 g of a liquid medium. To this solution, 1,000 g of an antisolvent are added. If the solubility in the resulting mixture comprising both antisolvent and liquid medium is, for example, 25 g per 1,000 g of said mixture, 50 g of the compound will solidify. If the solubility of the compound in said mixture of liquid medium and antisolvent had been 50 g per 1,000 g, no solute would have solidified, as the total amount of liquid media (2,000 g) would have been enough to dissolve 100 g of the compound.

It is noted that for practical purposes a gaseous or a supercritical antisolvent or preferably a liquid antisolvent is employed after the addition of 10 mol% of which, based on the amount of solvent(s) in the liquid medium, at 20°C to a liquid medium comprising a compound which is to be solidified, the solubility of said compound in the resulting liquid mixture is at least 50% less than in the liquid medium alone at 20°C.

The amount of compound to be solidified which can be dissolved in a liquid medium/antisolvent mixture cannot be predicted using the solubilities in the pure liquid medium and the pure antisolvent. As is known by the skilled person, this can be determined experimentally using liquid medium/antisolvent mixtures comprising different liquid medium/antisolvent ratios. The results of these experiments can be used to determine which liquid medium/antisolvent ratio gives the maximum yield.

Preferably, the antisolvent used in the process according to the present invention is environmentally harmless, inflammable, non-explosive, non-toxic, non-smelling, non-corrosive, chemically stable, easy to handle, easily available and/or inexpensive. The choice of antisolvent is very important for the product quality and the overall process economics. It was found that the use of COSMOTHERM^{®}, a Chemical Computational tool for calculating the chemical potential of compound(s) to be solidified-solvent systems will make the selection of suitable antisolvents easier due to improved chemical understanding.
Preferred antisolvents include alcohols, ketones, carboxylic acids, esters, ethers, alkanes, water, amines, (food grade) quaternary ammonium salts, ionic liquids, gaseous carbon dioxide, and supercritical liquids. Examples of particularly preferred antisolvents include water, methanol, ethanol, hexane, pentane, polyethylene glycol, choline chloride, ionic liquids comprising (metal) complexes of EDTA, and a ferric-gluconate-sucrose complex.

The liquid medium employed in the process according to the present invention can form a one-phase or a two-phase system after being mixed with the one or more antisolvents. However, preferably, the liquid medium/antisolvent mixture is a one-phase system. Said liquid medium comprises at least one organic or inorganic compound which is to be solidified and at least one solvent for said compound or compounds. The liquid medium can comprise two or more solvents but preferably comprises just one solvent. Such a solvent preferably is a liquid in which the compound to be solidified dissolves at least to a reasonable extent. In a preferred embodiment the concentration of the compound(s) to be solidified in the liquid medium is at least 0.1 g/l, more preferably at least 0.2 g/l and even more preferably at least 1 g/l. The optimum concentration of the compound(s) in the liquid medium, however, is dependent *int. al.* on the antisolvent(s) used, the properties of the compound(s) to be solidified, the desired purity of the solid composition, and in the case of a crystalline composition, the crystal size.
For example, if the saturation concentration of a compound in the liquid medium employed in the process according to the present invention is between about 0.1 g/l and about 1 g/l at 20°C, preferably, the concentration of the compound in the liquid medium is in the range of from 70-100 wt%, more preferably 95-100 wt% of the saturation concentration. For a compound with a solubility in the liquid medium at 20°C of between about 1 g/l and about 50 g/l, preferably a concentration in the range of 50-100 wt%, preferably 75-99.8 wt%, and most preferably 90-99 wt% of the saturation concentration is used. For a compound with a saturation concentration in the solvent at 20°C of about 50 g/l or more, the concentration of the compound in the liquid medium preferably is in the range of from 30-100 wt%, more preferably, 50-99 wt%.
It is noted that in an embodiment wherein the liquid medium comprising the compound(s) to be solidified is forced through a membrane into one or more antisolvents, preferably, the compound(s) to be solidified is/are dissolved in the liquid medium in a concentration of at most 99 wt% of the saturation concentration to avoid the risk of plugging of the membrane.

The liquid medium may in addition comprise conventional adjuvants such as surfactants, crystal growth inhibitors, additives to change the morphology of the crystals, additives for changing the modification of the crystals (*i.e.* the type of crystal lattice) and/or additives to avoid clustering of particles.

If the liquid medium forms a two-phase system with the one or more antisolvents employed in the process according to the present invention, preferably an emulsion or an emulsion-like mixture is formed wherein the one or more antisolvents form the continuous phase and the droplets of the liquid medium comprising the compound(s) to be solidified form the dispersed phase. It is also preferred in this embodiment to add emulsifiers to the antisolvent(s) and/or the liquid medium in order to stabilise the emulsion formed. For this purpose any conventional emulsifier or mixture of emulsifiers can be used.
A wide variation of solvent (i.e. in the liquid medium) and antisolvent combinations is possible. In a preferred embodiment the volume ratio between the solvent and the antisolvent may vary widely. For example, 20 volume parts of solvent may be combined with 1 part of antisolvent. However, the risks of agglomeration are advantageously reduced by choosing a ratio of volume parts of solvent to volume parts of antisolvent in the range from 5:1 to 1:20, or more preferably in the range from 1:1 to 1:10.
The optimum amount of antisolvent preferably used in order to obtain a maximum yield depends on the solidification properties of the compound to be solidified and its solubility in the antisolvent and the liquid medium. However, it can easily be determined by the skilled person by routine experimentation.
In a further preferred embodiment the solvent and the antisolvent are chosen such that the ratio of dissolved compound in a saturated solution of solvent at 20°C to dissolved compound in a saturated solution of antisolvent at 20°C lies in the range of 3:1 to 1,000,000:1, more preferably in the range of 50:1 to 10,000:1, and most preferably in the range of 100:1 to 1,000:1.

It is noted that the liquid medium and/or the one or more antisolvents may comprise one or more inert liquid compounds. Such inert compounds are typically denominated as nonsolvents. This term denotes a liquid medium which differs in chemical composition from the solvent and antisolvent(s) employed in the process according to the present invention, which does not separate from said liquid medium but, after being incorporated into the liquid medium, does not induce solidification of the compound. More precisely, when 1,000 g of a liquid which is a possible nonsolvent are mixed with 1,000 g of the solution of the compound to be solidified in the solvent to be used in the process according to the invention, the amount of compound which will dissolve in a saturated solution of said compound in the combined liquid media may not increase or decrease by more than 10 wt% compared to the amount of compound which will dissolve in a saturated solution of said compound in 2,000 g of the liquid medium, at a temperature of 20°C, for said liquid to be called a nonsolvent. Nonsolvents which have been added to the liquid medium and/or the antisolvent(s) normally serve as a carrier, e.g. as a diluent. By making use of nonsolvents, the solidification conditions with respect to hydrodynamics and concentrations can be tuned even more finely.
Furthermore, the liquid medium can contain a limited amount of one or more antisolvents and/or the one or more antisolvents can contain a limited amount of a solvent for the compound(s) to be solidified. The admixture of the one or more antisolvents to the liquid medium and/or the admixture of solvent to the one or more antisolvents can be used as a means to control the particle size of the particles formed. More precisely, the particle size of the compound(s) which is/are crystallized will decrease if an increasing amount of solvent is present in the antisolvent(s). However, the absolute particle size depends on the type(s) of solvent(s) and/or antisolvent(s) used. Furthermore, the liquid medium and/or antisolvent(s) may contain solid particles for various reasons such as seeding and may also contain traces of other liquids for various reasons. It should also be noted that the liquid medium may also be supercritical.

The term membrane as used throughout this specification can denote any conventional membrane having an average pore size up to 3 µm. Furthermore, the term membrane includes dense polymeric membranes such as pervaporation and reverse osmosis membranes. Preferably, however, use is made of conventional membranes selected from the group consisting of nano-filtration membranes (0.8 nm up to 9 nm pores), ultra-filtration membranes (3 nm up to 100 nm pores), and micro-filtration membranes (50 nm up to 3 µm pores).
Said membranes can have a shape selected from tubes, fibres, and, spiral wounds. Preferably, the membrane has a tubular shape. The pores of the membrane can have any kind of shape, including for example a round shape, square shape, slit shape or irregular shape. Preferably, the pores have a more or less round shape.
Said membrane is positioned inside a membrane module. By the term membrane module is meant a unit comprising one or more membranes positioned in between one or more inlets for the liquid medium and one or more inlets for the antisolvent(s). Furthermore, said membrane module comprises one or more outlets for the combined liquid media. The membrane(s) in the membrane module can be reinforced by a material such as ceramics, metals, polymers, etc. Furthermore, said membrane module can contain, for example, a sequential set-up of one or more membranes, optionally with different pore-sizes, or a set up wherein one or more membranes, optionally with different pore-sizes, are placed concentric with one another. Important classes of membranes are inorganic membranes, organic/inorganic membranes, and polymeric membranes. Said classes of membranes can be applied in the process according to the present invention, dependent on the liquid media and/or antisolvents used. The person skilled in the art can select the proper membrane on the basis of common general knowledge and the information disclosed herein.

As mentioned above, in the process according to the present invention, the membrane is used as a precision dosing device in order to achieve efficient micromixing of the liquid medium comprising at least one compound which is to be solidified and one or more antisolvents. By micromixing as used throughout this specification is meant that a (to be dispersed) liquid medium is forced through a membrane from one side while at the other side of the membrane a second liquid medium, *i.e.* the continuous phase, flows along the membrane surface area. It is envisaged that by doing so the (to be dispersed) liquid medium forms small droplets on the other side of the membrane, which at a certain moment emerge or dissolve into the continuous phase. If the liquid medium is completely miscible with the continuous phase, one should realise that the droplet is just an imaginary droplet (as no phase boundary will be visible). This imaginary droplet may be envisaged as a small liquid volume with the size and shape of a droplet that consists (mainly) of said liquid medium. The size of said droplets depends among others on the average pore size and/or the pore distribution of the membrane, the type of membrane, the (pulling) force of the flowing continuous phase, the pressure difference, the surface tension, and the contact angle with the membrane surface. The length scale on which the mixing takes place is estimated to be of the same order of magnitude as the length scale of the pores of the membranes. As these pores are most preferably smaller than 25 µm, mixing most preferably takes place on a very small scale. It is noted that it is also possible to force the (to be dispersed) liquid medium through the membrane into a stagnant liquid medium (*i*.*e*. dead-end dosing). Different dosing profiles can be used, for example by using fluctuating dosing rates, changing dosing rates along the length of the membrane, or sequentially adding an (inert) gas and liquid antisolvent(s) or liquid(s). Changing the dosing profiles, using a mixing device to improve the mixing of the liquid medium and the antisolvent(s), applying temperature differences between, for example, the liquid and the antisolvent or over the length of the membrane, and/or using conventional sonification methods are examples of techniques which can be applied in order to optimise the solidification process and the product quality of the solid particles thus obtained.

In a particularly preferred embodiment, a solution of a compound in a liquid medium is forced into antisolvent(s) via the pores of a membrane. The ratio between the antisolvent flow rate through the membrane module and the liquid medium flow rate is preferably larger than 1, or more preferably larger than 1.5. The preferred antisolvent and liquid medium flow rates are *int. al.* dependent on the type of membrane used, the size of the membrane, the liquid medium, and the antisolvent(s) and can easily be determined by the skilled person. It is noted that in a preferred embodiment, the morphology of the crystals, *i.e.* the shape of the crystals, can be altered by varying the flow of the antisolvent(s) and/or of the liquid medium.
In an embodiment wherein antisolvent(s) is/are forced via the pores of a membrane into a liquid medium comprising at least one organic or inorganic compound, the ratio between the liquid medium flow rate through the membrane module and the antisolvent flow rate preferably is between 1:1 and 1:10, more preferably between 1:1 and 1:5.

The liquid medium and the antisolvent(s) are mixed under either laminar flow conditions or turbulent flow conditions. Preferably, they are mixed under laminar flow conditions due to the predictable and uniform hydrodynamics. Preferably, within 24 hours, more preferably within 12 hours, even more preferably within 1 hour, yet more preferably within 15 min after mixing of the liquid medium and the antisolvent(s), the organic and/or inorganic compound(s) start to solidify. Most preferably, within 2 min after mixing, said compound(s) start(s) to solidify, which means that most preferably said compounds will solidify within the membrane module.

The characteristics of the liquid medium or emulsion formed after the one or more antisolvents have been mixed with the liquid medium comprising the compound(s) which is/are to be solidified can be influenced by selecting the proper membrane. For example, if the medium after mixing is an emulsion, a narrow droplet diameter distribution of the dispersed phase can be obtained by using a membrane with a narrow pore size distribution as the dosing unit.

During and/or after the solidification process the generated solid particles, preferably crystalline particles, may, if so desired, be separated from the remaining liquids by any conventional method of solid/liquid (S/L) separation. Preferably, the crystalline particles are recovered by (micro)filtration, methods based on density difference, such as centrifugation, or by sedimentation. Due to the narrow crystal size distribution and the uniform particle shape of the crystallised compounds obtained with the process according to the present invention, in most cases less plugging of the filter or loss of product will occur. Thus, in general, with the antisolvent crystallisation process according to the present invention S/L separation will become easier compared to the filtration of products obtained with conventional crystallisation processes.

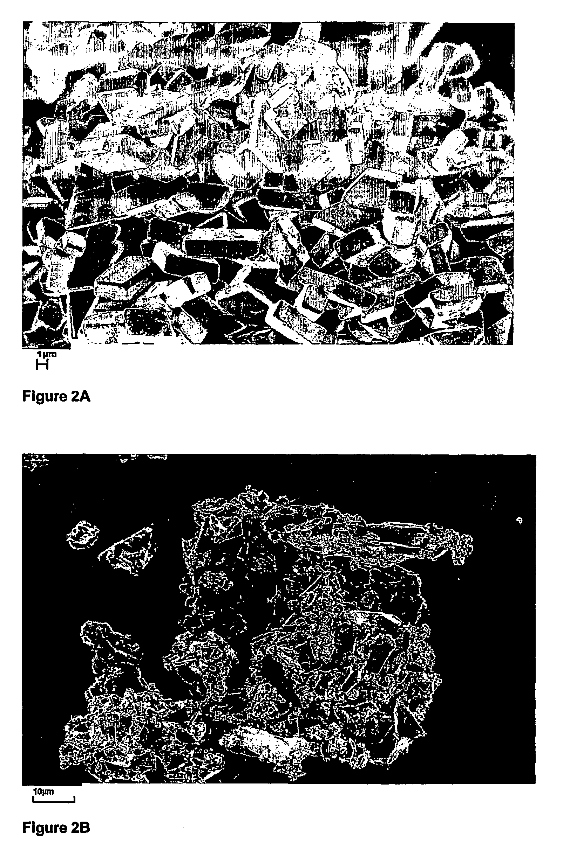

The process according to the invention results in a composition comprising solid particles wherein the particles show only little and preferably essentially no agglomeration. Furthermore, the process according to the invention results in a solid composition comprising particles having a smoother surface than the particles obtained with prior art processes (see for example figures 2A and 2B).

The process as described above results in solid, preferably, crystalline particles which advantageously have a high purity and, furthermore, a narrow particle size range. The span, defined as (d₉₀-d₁₀)/d₅₀, wherein d50, d10, and d90 can be understood to mean that 50%, 10%, and 90%, respectively, of the particles having a particle size smaller than or equal to the indicated value as determined by conventional laser diffraction technique, can even be below 1.4, which is exceptional for a solidification process such as a crystallisation process. In general, for the process according to the present invention the span preferably is below 3. More preferably, the span is below 2.5, and most preferably below 2.

Many of the current crystallisation processes are batch processes. This means that crystallisation and S/L filtration are sequential processes. In general, this requires bigger S/L separation devices, compared with continuous processes in which crystallisation and S/L separation occur simultaneously at comparable production capacities. The process according to the present invention is operated continuously. With the new, continuous, crystallisation process, in principle relatively small S/L separation units can be used. An additional advantage of operating in a continuous mode is that the flow ratio between the liquid medium comprising the compound to be crystallised and the one or more antisolvents can be controlled very well. Preferably, this ratio can also control the polymorphic structure of the crystalline composition formed. Most preferably, the crystallisation process according to the present invention is performed in such a way that after drying of the product no powder handling steps like milling and sieving are necessary.
In a particularly preferred embodiment according to the present invention, after recovery of the generated solid, and preferably crystalline, particles, the employed liquid medium and the one or more antisolvents are separated using a separating means such as a nanofiltration membrane to allow recycling of the antisolvent and/or liquid medium.

Scaling up of conventional antisolvent crystallisation processes wherein crystalline compositions are formed is often hard to achieve, because the use of larger equipment has a great influence on for example the hydrodynamics and the supersaturation phenomena. Hence, optimisation of the crystallisation processes generally has to be performed at the production site. By using the process according to the present invention, however, scaling up of the process can be achieved relatively easily due to the fact that scale-up is obtained by multiplication of the same membrane modules as were used for small-scale experiments in a parallel arrangement or by increasing the number of membranes in one module. Accordingly, the solidification, preferably crystallisation, process according to the present invention can be scaled up without any occurrence of the above-described problems.
This invention therefore also provides an arrangement of parallel antisolvent solidification modules, or in a specific embodiment, antisolvent crystallisation modules, wherein each such module comprises one or more, preferably tubular or capillary, membranes. Preferably, an arrangement is applied comprising between 2 and 30,000 parallel antisolvent solidification modules, more preferably between 5 and 5,000 parallel antisolvent solidification modules. For practical reasons an arrangement of 5 to 1,000 modules is preferred. Each module can comprise for example from 1 to 30,000 membranes, more preferably from 1 to 15,000 membranes. In a preferred embodiment two or more tubular membranes are placed in a parallel set-up in a solidification module, preferably a crystallisation module, in the shape of a vessel.

As mentioned above, the liquid medium and/or the one or more antisolvents can be recovered to allow the creation of a continuous, industrially useful process. In respect of this, antisolvents that form a two-phase system with the liquid medium are suitable. These antisolvents can be (partly) recovered from a spent mother liquor by increasing or decreasing its temperature to a value where the mutual solubilities of the antisolvent and the liquid medium are low, thus creating a two-phase system in which the two liquids can be easily separated from each other by conventional techniques.

As indicated above, the solidification process according to the present invention can be carried out either in a one-phase or in a two-phase system. In the one-phase system, the compound(s) present in the liquid medium will solidify because of the presence of the antisolvent that reduces the solubility of the compound(s) by binding the liquid medium. In the two-phase system, the driving force for solidification is created by the extraction of liquid medium into the antisolvent phase, and by the dissolution of the antisolvent in the liquid medium.
In addition to the above, use can be made of a difference in temperature. For example, when dissolving the organic or inorganic compound in a suitable solvent, the temperature can be increased, resulting in a liquid medium of elevated temperature. By mixing said liquid medium with an antisolvent of a lower temperature, a mixture of a lower temperature can be obtained. Such lowering of the temperature can be advantageous in view of the speed and yield of the process. The process can also be performed using an antisolvent with elevated temperature and a liquid medium of lower temperature.

In a further preferred embodiment of the present invention, the solid particles obtained by the addition of one or more antisolvents to a liquid medium in which the compound(s) was/were present, or vice versa, are encapsulated after their precipitation or crystallisation or simultaneously with their solidification. It is also possible, although less preferred, that if an emulsion is formed after the addition of the antisolvent to the liquid medium, or vice versa, capsules are generated containing a liquid core and a solid shell material comprising the compound(s) which was/were to be solidified.

Encapsulation is a technique frequently used to generate capsules that typically contain a liquid core material and a shell material that brings consistency to the particle. However, these techniques can also be used to encapsulate preformed particles via the process according to the present invention. The encapsulation can for instance modify the colour, shape, volume, apparent density, reactivity, durability, pressure sensitivity, heat sensitivity, and photosensitivity of the encapsulated compound(s). Encapsulated particles have many useful functions and have been employed in many different areas, frequently connected with applications in which the contents of the capsule have to be released into the surrounding environment under controlled conditions. By encapsulating compounds which are solidified, it is for example possible to increase the storage life of a volatile compound. Further, the core material in encapsulated compounds can be protected from the effects of UV rays, moisture, and oxygen. Chemical reactions between two active species can be prevented by physical separation due to the encapsulation and finally, finely divided powders can be encapsulated to reduce agglomeration problems.
The shell material used for the encapsulation preferably is of a synthetic nature such as polymeric materials, but also materials such as gelatine and alginate can be used. Said material and also the manner of encapsulation can be easily selected by the skilled person on the basis of the physical properties of the compound(s) to be encapsulated and the intended application.

In addition the process according to the invention can be used to coat particles themselves. These can be particles prepared according to the invention or particles prepared in some other, conventional manner.
Hence this invention also provides a process as described above, wherein a solid composition of solid particles is prepared, in which composition at least part of the particles consists of a core coated with one or more solid coatings of one or more organic or inorganic coating materials, by forcing a liquid medium comprising dissolved organic or inorganic coating material through a membrane into a suspension of particles to be coated in one or more antisolvent(s) for said coating material.

In a preferred embodiment both the particles to be coated and the coated particles are prepared by the process according to the invention. In this manner only one coating or two or more coatings can be applied.
In such a process
a) a first liquid medium comprising at least one dissolved organic or inorganic compound is forced through a membrane into one or more antisolvents or one or more antisolvents are forced through a membrane into a liquid medium comprising at least one organic or inorganic compound, yielding a composition comprising solid particles comprising said organic and/or inorganic compound(s);
b) whereafter in a further step at least part of the prepared solid particles is coated with one or more solid coating(s) of a coating material by forcing a second liquid medium, with a coating material dissolved therein, through a membrane into a suspension of said solid particles in an antisolvent for said coating material, yielding a composition comprising coated solid particles.
Such a process can advantageously be carried out by a sequential set-up of one or more membrane modules or a set-up wherein one or more membranes are placed concentric with one another in one membrane module. In the sequential set-up of membrane modules a core of a particle can be prepared in a first membrane module, yielding solid particles, which can be coated in a subsequent, e.g. second, membrane module. The particles yielded by a previous membrane module can be generated as a slurry or suspension, which can be forwarded directly to the next membrane module. In a preferred embodiment, however, the solid particles obtained from a previous membrane module are separated from the liquid mixture and optionally purified before they are fed into the next membrane module.

In a further embodiment at least the core or one or more coatings comprise a pharmaceutical compound. In a further preferred embodiment the core comprises a first pharmaceutical compound while at least one coating comprises a second, different pharmaceutical compound.

The coating material(s) can be any desired type of coating material, including for example polymeric material, antifungal preservatives, antimicrobial preservatives, anti-oxidants, emulsifiers, flavourants, sweeteners, surfactants or another active compound. Examples of suitable materials for the coating include but are not limited to gelatin, pectin, polyethylene glycols, alginate, polyvinyl acetate, polyvinyl chloride, polyethyleneoxide co-polymers, trometamol, hydroxypropyl methyl cellulose, hydroxypropyl cellulose, hydroxyethyl cellulose, methyl cellulose (e.g. methocel), ethyl cellulosegelatin (e.g. ethocel), cellulose acetate phthalate, shellac, aspartame, dextrose, mannitol, sorbitol, sucrose, ascorbic acid, ascorbyl palmitate, butylated hydroxyanisole, butylated hydroxytoluene, hypophosphorous acid, sodium ascorbate, sodium bisulfide sodium formaldehyde sulfoxylate, sodium metabisulfite, hypophosphorous acid, propyl gallate, monothioglycerol, acacia, cetomacrogol, cetyl alcohol, glyceryl monostearate, sorbitan monooleate, benzalkonium chloride, polysorbates, sodium lauryl sulfate, sorbitan and/or monopalmitate.

In another embodiment according to this invention, one can also encapsulate a particle (or one could also define it as coating) by modifying the surface properties as a post-treatment of the previously formed solid particles. A modification (for instance via chemical reactions) of the particle surface can generate a shell material with new added functionalities. One could think of for instance oxidation of the surface. This way properties such as hardness, solubility, and shape can be modified and tuned at will.

In one special embodiment both the core and one or more coating materials are pharmaceutically active compounds, such as to prepare particles comprising two pharmaceutically active compounds in a well defined ratio. Such particles can be wholly or partly crystalline.
In a further embodiment particles for use in a product with a slow release of a pharmaceutically active compound are prepared. Apart from the antisolvent for the coating material, the suspension can also comprise one or more nonsolvents as described hereinbefore.

The process as described hereinbefore can advantageously be used to prepare a pharmaceutical dosage form in which the active ingredient is distributed in an advantageously homogeneous manner. Because of the small span and little variation in particle size, the process furthermore is especially advantageous for use in the preparation of pharmaceutical products for inhalation. In one special embodiment, therefore, such a pharmaceutical dosage form is a product for inhalation. In another embodiment such a pharmaceutical dosage form is a tablet.

The present invention is elucidated by means of the following non-limiting Examples.

### EXAMPLE 1: Production of a crystalline NaCl composition

The following procedure was applied for the production of sodium chloride crystals using the antisolvent crystallisation process according to the present invention. All experiments were performed at ambient conditions. The resulting crystals were analysed by SEM (Scanning Electron Microscopy) and CSD (Crystal Size Distribution) measurements by means of laser diffraction using a Mastersizer 2000 apparatus of Malvern Instruments with a Fraunhofer model for data analysis.

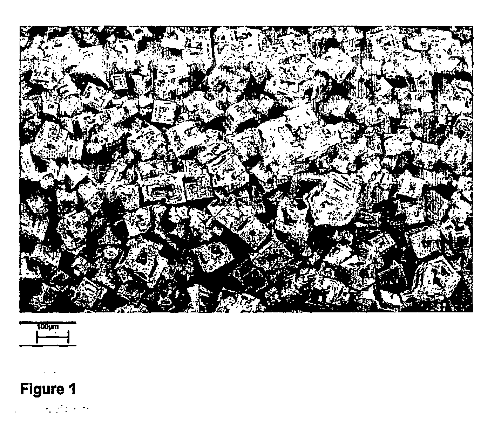

A 25wt% sodium chloride solution was prepared at room temperature. Pure ethanol was used as an antisolvent. Said salt solution was dosed from the outside of a tubular membrane into the antisolvent (ethanol) on the inside of this membrane. Said membrane was a hydrophobic tubular SPG^{®}-membrane, type UP11023, with a pore-size of 1.1 µm and with a 10 mm inner diameter. A gear pump was used to control the velocities. The speed of the antisolvent was set to 50 l/hr and the flow of the salt-solution was set to 60 l/hr.
The crystals thus obtained were collected using filtration, washed with 100% ethanol, and subsequently dried in an oven. Figure 1 shows a SEM picture of the obtained crystals. The CSD measurement showed an average crystal size d50 of 40 µm and a span of 1.5.

An additional experiment was performed in which a simple stirring rod (25.5 cm in length and 3 mm in diameter, having 8 blades on the rod each having a length of 15 mm and a width of 2 mm) was mounted inside the hydrophobic tubular SPG^{®}-membrane, type UP11023 (supplier: SPG Technology Co. Japan) to provide additional macromixing within the membrane. The rotation speed of the stirrer was set to 630 rpm. Again, a salt solution of 25 wt% was used and ethanol was used as the antisolvent. The salt solution was dosed to the antisolvent using the above-mentioned membrane. The flow of the salt solution was set to 40 l/hr and the flow of the ethanol to 50 l/hr. The resulting crystals were collected using filtration and washed with 100% ethanol. Said crystals had a span of 1.2.

### EXAMPLE 2: Production of a crystalline 3-ketodesogestrel composition

The following procedure was applied for the production of 3-ketodesogestrel using the antisolvent crystallisation of the present invention. All experiments were performed at ambient conditions. The resulting crystals were analysed using CSD measurements that were measured via conventional laser diffraction technique.

A 3-ketodesogestrel solution was prepared of 97 g 3-ketodesogestrel in 5 I of ethanol. Water was used as antisolvent. For dosing, a Microdyn^{®} type SE020TP1 N membrane with an average pore size of 1 µm was used. The 3-ketodesogestrel solution was dosed from the outside of the membrane to the antisolvent on the inside of the membrane. The speed of the antisolvent was set to 45 l/hr. The speed of the 3-ketodesogestrel flow was set to 0.41 l/hr. Again, a gear pump was used to control the velocities. The crystals thus obtained were collected using filtration, washed with 100% ethanol, and subsequently dried in an oven. Figure 2A shows a SEM picture of the obtained crystals. CSD measurement by means of laser diffraction using a Mastersizer 2000 apparatus of Malvern Instruments with a Fraunhofer model for data analysis showed a d10 value of 3 µm, d50 = 10 µm, and d90 = 20 µm. The span was determined to be 1.7. Herein d50, d10; and d90 can be understood to mean that 50%, 10%, and 90%, respectively, of the particles have a particle size smaller than or equal to the indicated value.

The above-described experiment was repeated using different flow rates for the liquid medium flow and the antisolvent flow in order to investigate their influence on the particle size distribution.
Table 1 summarises the different flow rates used and the CSD values obtained.

**Table 1. Experiments with 3-ketodesogestrel using a Microdyn^{®} membrane with pore size 1 µm**

| **Entry** | **antisolvent flow [l/h]** | **3-ketodesogestrel flow [l/h]** | **d10** | **d50** | **d90** | **Span** |
|---|---|---|---|---|---|---|
| **1** | 45 | 0.7 | 3 | 11 | 21 | 1.6 |
| **2** | 45 | 0.4 | 3 | 10 | 20 | 1.7 |
| **3** | 45 | 6.2 | 5 | 16 | 45 | 2.5 |
| **4** | 90 | 0.7 | 4 | 10 | 23 | 1.9 |
| **5** | 90 | 5.0 | 2 | 7 | 22 | 2.9 |
| **6** | 45 | 10 | 2 | 8 | 24 | 2.8 |
| **7** | 45 | 42 | 12 | 42 | 90 | 1.9 |
| **8** | 45 | 20 | 2 | 9 | 29 | 3 |

### COMPARATVE EXAMPLE 3

10 kg of 3-ketodesogestrel was dissolved in 200 litres of ethanol. This mixture was treated with 400 g of activated carbon and subsequently filtered. The filtrate was reduced to a volume of 80 litres by heating to 90°C. 105 litres of water were added to the solution at ambient temperature and the mixture was subsequently cooled to 0°C. The mixture was stirred for 2 hours at 0-2°C. The resulting crystals were filtered off and subsequently dried in vacuum at a temperature of 50°C maximum until a water content of >0.4% was reached. The resulting crystals were micronised at a rate of 15 kg per hour in a 20 cm self-built spiral-stream jet mill equipped with nozzles. The dosing pressure was 3 bar, the milling pressure was 7 bar.
Figure 2B shows a SEM picture of the obtained crystals.
From comparing Figure 2A with Figure 2B it can be observed that the particles obtained by the process according to the present invention have a smoother surface and a significantly smaller span than the particles obtained according to a conventional process.

### EXAMPLE 4: Production of a crystalline progesteron composition

The following procedure was applied for the production of progesteron. All experiments were performed at ambient conditions. The resulting crystals were analysed by CSD measurements by means of laser diffraction using a Mastersizer 2000 apparatus of Malvern Instruments with a Fraunhofer model for data analysis.

A progesteron solution was prepared of 50 g of progesteron per litre of ethanol. Water/ethanol mixtures of different compositions (See Table 2) were used as antisolvent. For dosing, a Microdyn^{®} type SE020TP1 N membrane with an average pore size of 1 µm was used. The progesteron solution was dosed from the outside of the membrane to the antisolvent on the inside of the membrane. A gear pump was used to control the flow velocities. The speed of the antisolvent was set to 45 l/h and the speed of the progesteron solution was set to 12 l/h. The crystals thus obtained were collected using filtration, washed with 100% ethanol, and subsequently dried in an oven.

Table 2 summarises the different antisolvent compositions used and the obtained CSD results. As can be derived from said Table, in all cases crystalline progesterone particles with a surprisingly small span were obtained.

**Table 2. Experiments with progesteron using a Microdyn^{®} membrane with pore size 1µm**

| **Entry** | **wt% water** | **wt% ethanol** | **d[10]** | **d[50]** | **d[90]** | **Span** |
|---|---|---|---|---|---|---|
| **1** | 100 | 0 | 2 | 7 | 16 | 2 |
| **2** | 85 | 15 | 4 | 12 | 25 | 1.8 |
| **3** | 70 | 30 | 7 | 21 | 43 | 1.7 |

### EXAMPLE 5: Production of a crystalline tibolone composition

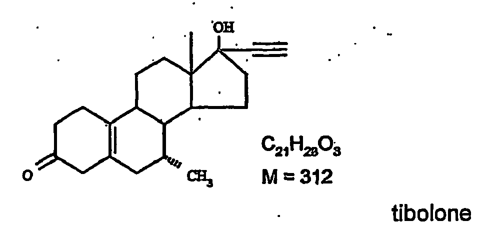

As a further example tibolone (see the above structure) was crystallised by a process wherein 34 mg of tibolone were dissolved in 1 kg of ethanol. As an antisolvent a mixture of 90 wt% water and 10 wt% ethanol was used. The tibolone-ethanol solution was pressed into the antisolvent through a tubular hydrophobic SPG^{®} membrane, type UP11023 (supplier: SPG Technology Co. Japan) with a pore size of 1.1 µm with a 10 mm inner diameter. The flow rate of the tibolone solution was 65 l/hr, while the flowrate of the antisolvent was 15l/hr. Crystalline particles with a d50 = 18.2 µm, d10 = 3.8 µm, and d90 = 51.3 µm were obtained.

## Claims

1. Antisolvent solidification process for preparing a solid composition comprising at least one organic or inorganic compound, wherein a liquid medium comprising at least one dissolved organic or inorganic compound is forced through a membrane which is positioned in a membrane module into one or more antisolvents or wherein one or more antisolvents are forced through a membrane which is positioned in a membrane module into a liquid medium comprising at least one organic or inorganic compound, whereby the process is carried out as a continuous process, and whereby the membrane has up to 3µm pores and the shape of the membrane is selected from tubes, fibres or spiral wounds, yielding a composition comprising solid particles comprising said organic and/or inorganic compound(s).

2. A process according to claim 1 wherein the solidification is a crystallisation, the prepared solid particles are crystalline particles, the organic or inorganic compound is a crystallisable compound, and, optionally, said crystalline particles are recovered from the process.

3. A process according to any one of claims 1-2 wherein the liquid medium is separated from the one or more antisolvents by means of nanofiltration and wherein, optionally, the liquid medium and/or the antisolvent(s) is/are recycled.

4. A process according to any one of claims 1-3 wherein an emulsion is formed before said composition comprising solid particles is obtained.

5. A process according to any one of claims 1-4 wherein a nonsolvent is present in the liquid medium and/or in the one or more antisolvents.

6. A process according to any one of claims 1-5 wherein the organic or inorganic compound is selected from the group consisting of transition metal compounds, transition metal salts, alkali salts, alkali earth salts, fatty acids, proteins, saccharides, aminoacids, and pigments.

7. A process according to any one of claims 1-6 wherein the solid particles essentially consist of particles of only one inorganic or organic compound.

8. A process according to any one of claims 1-7 wherein the inorganic or organic compound is a pharmaceutical compound.

9. A process according to claim 8 wherein the pharmaceutical compound is selected from the group consisting of tibolone, progesterone, desogestrel, and 3-keto-desogestrel (etonogestrel).

10. A process according to any one of claims 1-7 wherein the solid composition comprises a mixture of two or more pharmaceutical compounds.

11. A process according to any one of claims 1-3 wherein a composition comprising solid particles is prepared, in which composition at least part of the particles consists of a core coated with one or more solid coatings of one or more organic or inorganic coating materials, by forcing a liquid medium comprising dissolved organic or inorganic coating material through a membrane into a suspension of particles to be coated in one or more antisolvent(s) for said coating material.

12. A process according to claim 11 wherein the prepared solid composition comprises particles having a core comprising a pharmaceutical compound coated with at least one or more coating materials which comprise a pharmaceutical compound.

13. Use of the process according to any one of claims 1-12 in the preparation of a pharmaceutical dosage form.

14. Use according to claim 13 wherein the dosage form is a tablet.

15. Use according to claim 13 wherein the dosage form is a product for inhalation.

## Patentansprüche

1. Verfahren zur Verfestigung mit einem Anti-Lösungsmittel zur Herstellung einer festen Zusammensetzung umfassend mindestens eine organische oder anorganische Verbindung, wobei ein flüssiges Medium, umfassend mindestens eine gelöste organische oder anorganische Verbindung, durch eine Membran hindurch, welche innerhalb eines Membran-Moduls angeordnet ist, in eines oder mehrere Anti-Lösungsmittel gezwungen wird oder wobei eines oder mehrere Anti-Lösungsmittel durch eine Membran hindurch, welche innerhalb eines Membran-Moduls angeordnet ist, in ein flüssiges Medium gezwungen werden, umfassend mindestens eine organische oder anorganische Verbindung, wobei das Verfahren als ein kontinuierliches Verfahren ausgeführt wird und wobei die Membran bis zu 3µm Poren aufweist und die Gestalt der Membran ausgewählt ist aus Röhren, Fasern oder Spiralwindungen, ergebend eine Zusammensetzung umfassend feste Partikel umfassend die besagten organischen und/oder anorganischen Verbindung(en).

2. Verfahren nach Anspruch 1, bei dem die Verfestigung eine Kristallisation ist, wobei die hergestellten festen Partikel kristalline Partikel sind, die organische oder anorganische Verbindung eine kristallisierbare Verbindung ist, und, optional, die besagten kristallinen Partikel von dem Prozess wiedergewonnen werden.

3. Verfahren nach einem der Ansprüche 1 bis 2, bei dem das flüssige Medium von dem einen oder mehreren Anti-Lösungsmitteln durch Mittel einer Nano-Filtrierung getrennt wird, und wobei, optional, das flüssige Medium und/oder die Anti-Lösungsmittel wiedergewonnen werden. Anti-Lösungsmittel wiedergewonnen werden.

4. Verfahren nach einem der Ansprüche 1 bis 3, bei dem eine Emulsion ausgebildet wird, bevor die besagte Zusammensetzung, die die besagten festen Partikel umfasst, erhalten wird.

5. Verfahren nach einem der Ansprüche 1 bis 4, bei dem ein Nicht-Lösungsmittel in dem flüssigen Medium und/oder in einem oder mehreren Anti-Lösungsmitteln vorhanden ist.

6. Verfahren nach einem der Ansprüche 1 bis 5, bei dem die organische oder anorganische Verbindung ausgewählt ist aus der Gruppe bestehend aus Übergangsmetall-Verbindungen, Übergangsmetall-Salzen, Alkalisalzen, Erdalkalisalzen, Fettsäuren, Proteinen, Sacchariden, Aminosäuren und Pigmenten.

7. Verfahren nach einem der Ansprüche 1 bis 6, bei dem die festen Partikel im Wesentlichen aus Partikeln aus nur einer anorganischen oder organischen Verbindung bestehen.

8. Verfahren nach einem der Ansprüche 1 bis 7, bei dem die anorganische oder organische Verbindung eine pharmazeutische Verbindung ist.

9. Verfahren nach Anspruch 8, bei dem die pharmazeutische Verbindung ausgewählt ist aus der Gruppe bestehend aus Tibolon, Progesteron, Desogestrel, und 3-Keto-Desogestrel (Etonogestrel).

10. Verfahren nach einem der Ansprüche 1 bis 7, bei dem die feste Zusammensetzung eine Mischung aus zwei oder mehr pharmazeutischen Verbindungen umfasst.

11. Verfahren gemäss einem der Ansprüche 1 bis 3, beim dem eine die festen Partikel umfassende Zusammensetzung, in welcher Zusammensetzung mindestens ein Teil der Partikel aus einem Kern bestehen, welcher mit einem oder mehreren festen Beschichtungen von einem oder mehreren organischen oder anorganischen Beschichtungsmaterialien beschichtet ist, durch das Hindurchzwingen eines flüssigen Mediums umfassend gelöstes organisches oder anorganisches Beschichtungsmaterial durch eine Membran in eine Suspension von Partikeln hergestellt wird, die in einem oder mehreren Anti-Lösungsmitteln für das besagte Beschichtungsmaterial zu beschichten sind.

12. Verfahren nach Anspruch 11, bei dem die hergestellte feste Zusammensetzung Partikel umfasst, die einen Kern haben, der eine pharmazeutische Verbindung umfasst, die mit mindestens einem oder mehreren Beschichtungsmaterialien beschichtet ist, welche eine pharmazeutische Verbindung umfassen.

13. Verwendung des Verfahrens gemäss einem der Ansprüche 1 bis 12 bei der Herstellung einer pharmazeutischen Dosierungsform.

14. Verwendung gemäss Anspruch 13, bei dem die Dosierungsform eine Tablette ist.

15. Verwendung gemäss Anspruch 13, bei dem die Dosierungsform ein Produkt zum Inhalieren ist.

## Revendications

1. Procédé de solidification d'antisolvant pour préparer une composition solide comprenant au moins un composé organique ou inorganique, dans lequel un milieu liquide comprenant au moins un composé organique ou inorganique dissous est passé à travers une membrane, qui est positionnée dans un module de membrane dans un ou plusieurs antisolvants, ou dans lequel un ou plusieurs antisolvants sont passés à travers une membrane qui est positionnée dans un module de membrane dans un milieu liquide comprenant au moins un composé organique ou inorganique, où le procédé est effectué en tant que procédé continu et où la membrane possède des pores jusqu'à 3 µm, et la forme de la membrane est choisie parmi des tubes, des fibres ou des enroulements en spirale, produisant une composition comprenant des particules solides comprenant le ou lesdits composants organiques et/ou inorganiques.

2. Un procédé selon la revendication 1, dans lequel la solidification est une cristallisation, les particules solides préparées sont des particules cristallines, le composé organique ou inorganique est un composé cristallisable et, éventuellement, lesdites particules cristallines sont récupérées depuis le procédé.

3. Un procédé selon l'une quelconque des revendications 1 et 2, dans lequel le milieu liquide est séparé du ou des antisolvants au moyen de nanofiltration, et dans lequel, éventuellement, le milieu liquide et/ou le ou les antisolvants sont recyclés.

4. Un procédé selon l'une quelconque des revendications 1 à 3, dans lequel une émulsion est formée avant que soit obtenue ladite composition comprenant des particules solides.

5. Un procédé selon l'une quelconque des revendications 1 à 4, dans lequel un non-solvant est présent dans le milieu liquide et/ou dans le ou les antisolvants.

6. Un procédé selon l'une quelconque des revendications 1 à 5, dans lequel le composé organique ou inorganique est choisi dans le groupe constitué de composés de métaux de transition, de sels de métaux de transition, de sels alcalins, de sels alcalino-terreux, d'acides gras, de protéines, de saccharides, d'acides aminés et de pigments.

7. Un procédé selon l'une quelconque des revendications 1 à 6, dans lequel les particules solides comprennent essentiellement des particules d'un seul composé inorganique ou organique.

8. Un procédé selon l'une quelconque des revendications 1 à 7, dans lequel le composé inorganique ou organique est un composé pharmaceutique.

9. Un procédé selon la revendication 8, dans lequel le composé pharmaceutique est choisi dans le groupe constitué de tibolone, de progestérone, de désogestrel et de 3-céto-désogestrel (étonogestrel).

10. Un procédé selon l'une quelconque des revendications 1 à 7, dans lequel la composition solide comprend un mélange de deux composés pharmaceutiques ou plus.

11. Un procédé selon l'une quelconque des revendications 1 à 3, dans lequel est préparée une composition comprenant des particules solides, dans laquelle au moins une partie des particules comprend un noyau recouvert d'un ou plusieurs revêtements solides d'un ou plusieurs matériaux de revêtement organiques ou inorganiques, en faisant passer un milieu liquide comprenant un matériau de revêtement organique ou inorganique dissous, à travers une membrane vers une suspension de particules à recouvrir, dans un ou plusieurs antisolvants pour ledit matériau de revêtement.

12. Un procédé selon la revendication 11, dans lequel la composition solide préparée comprend des particules possédant un noyau comprenant un composé pharmaceutique recouvert d'au moins un ou plusieurs matériaux qui comprennent un composé pharmaceutique.

13. Utilisation du procédé selon l'une quelconque des revendications 1 à 12, dans la préparation d'une forme pharmaceutique.

14. Utilisation selon la revendication 13, dans lequel la forme pharmaceutique est un comprimé.

15. Utilisation selon la revendication 13, dans lequel la forme pharmaceutique est un produit destiné à être inhalé.
